# EUROPEAN PATENT APPLICATION

(11) **EP 1 724 255 A1**
(43) Date of publication of application: **22.11.2006**
(21) Application number: 05720172.5
(22) Date of filing: 07.03.2005
(51) Int. Cl.: C07C 253/30, C07B 53/00, C07B 57/00, C07C 255/47, C12P 41/00

(54) **PROCESS FOR PRODUCTION OF CYCLOPENTENENITRILE DERIVATIVES**

(30) Priority: 09.03.2004 JP 2004065759
(71) Applicant: Japan Science and Technology Agency, Kawaguchi-shi, Saitama 332-0012 (JP)
(72) Inventor: MIYASHITA, Masaaki, Sapporo-shi, Hokkaido 0630842 (JP); TANINO, Keiji, Kita-ku, Sapporo-shi, Hokkaido 0010018 (JP); TOMATA, Yoshihide, Kita-ku, Sapporo-shi, Hokkaido 0010011 (JP); SHIINA, Yasuhiro, Ebetsu-shi, Hokkaido 0670041 (JP)
(74) Representative: Albrecht, Thomas
(86) International application number: PCT/JP2005/003900
(87) International publication number: WO 2005/085184

(57) **Abstract**

[PROBLEMS] To provide a process for the synthesis of cyclopentenenitrile derivatives through conjugate addition of a nitrile derivative to an α,β-unsaturated ketone whereby cyclopentenenitrile derivatives, particularly those capable of optical resolution, can be obtained in high yield without expensive reagents or compounds in a decreased number of steps.

[MEANS FOR SOLVING PROBLEMS] A novel process for the production of cyclopentenenitrile derivatives in high yield, which comprises trapping a compound obtained by conjugate addition of a 4-alkoxy-3-butenenitrile which may have alkyl or allyl at the α-position (e.g., 4-methoxy-3-butenenitrile) to an α,β-unsaturated ketone as an enol ester with acetic anhydride, chlorocarbonic ester, or the like and treating the enol ester with a strong acid or the like to form a five-membered ring.

## Description

### Technical Field

The present invention relates to a novel process for production of cyclopentenenitrile derivatives utilizing conjugate addition reaction to an α,β-unsaturated ketone.

### Background Art

Various processes for the synthesis of cyclopentene derivatives utilizing conjugate addition reaction to an α,β-unsaturated ketone have been known. However, since every process requires expensive reagents or compounds in addition to a number of steps, any process has scarcely been industrialized.
A process for the synthesis of cyclopentenenitrile derivatives utilizing conjugate addition reaction of nitrile derivatives has not been known at all until now.
Further, a process for industrial production of optically active cyclopentenenitrile derivatives has not been known at all.
In the reaction of a nitrile carbanion to an α, β-unsaturated ketone, generally only 1,2-addition reaction to the ketone occurs, and conjugate addition reaction does not occur.
Conventionally, an example of the reaction where conjugate addition is known to take precedence is limited to a reaction of substrate, which has an aryl substituent in the α-position as in phenylacetonitrile (see Non-Patent Document 1 and Non-Patent Document 2).

Non-Patent Document 1: R. Sauvetre, M.C. Roux, and J. Seyden-Penne, Tetrahedron, 34, 2135 (1978).
Non-Patent Document 2: M.C. Roux, L. Wartski, and J. Seyden-Penne, Tetrahedron, 37, 1927 (1981).

### Disclosure of Invention

### Problem to be Solved by the Invention

The present invention was made in consideration of such circumstances described above, and an object of the present invention is to provide a process for the synthesis of cyclopentenenitrile derivatives utilizing conjugate addition reaction of a nitrile derivative to an α,β-unsaturated ketone, whereby cyclopentenenitrile derivatives, particularly those capable of optical resolution, more specifically, those having a methyl group of the same stereo configuration as in the steroid skeleton at the bridge head, can be obtained in high yield without expensive reagents or compounds in a decreased number of steps.

### Means for Solving the Problem

The present invention relates to a process for production of cyclopentenenitrile derivatives, characterized in that an α,β-unsaturated ketone and a γ-alkoxy-β,γ-unsaturated nitrile are used as starting materials. More specifically, the present invention relates to a process for stereospecific production of cyclopentenenitrile derivatives, characterized in that an α,β-unsaturated ketone and a 4-alkoxy-3-butenenitrile which may have an alkyl group or an allyl group at the α-position are used as starting materials.
Further, the present invention relates to a process for production of cyclopentenenitrile derivatives, characterized by comprising obtaining a compound by performing conjugate addition reaction of an α,β-unsaturated ketone and a γ-alkoxy-β,γ-unsaturated nitrile, and subjecting said compound to a reaction to form a five-membered ring.
More specifically, the present invention relates to a process for production of cyclopentenenitrile derivatives, characterized by comprising obtaining a compound by performing conjugate addition reaction of an α,β-unsaturated ketone and a 4-alkoxy-3-butenenitrile which may have an alkyl group or an allyl group at the α-position, and converting said compound to an enol ester, and thereafter subjecting said enol ester to a reaction to form a five-membered ring.
Still further, the present invention relates to a process for production of optically active alcohol derivatives, comprising producing a cyclopentenenitrile derivative by the above-described process; reducing a keto group of said cyclopentenenitrile derivative to obtain a corresponding alcohol derivative; and thereafter subjecting the alcohol derivative to optical resolution.

Further more specifically, the present invention relates to a novel process for production of cyclopentenenitrile derivatives in high yield, characterized by comprising; (1)obtaining a compound by performing conjugate addition reaction of an α,β-unsaturated ketone and a 4-alkoxy-3-butenenitrile which may have an alkyl group or an allyl group at the α-position, e.g. 4-methoxy-3-butenenitrile, (2) converting said compound to an enol ester using an estrifying agent such as a derivative of organic carboxylic acid or carbonic acid, preferably, for example, acetic anhydride or a chlorocarbonate, and thereafter (3)treating said enol ester with, for example, a strong acid to form a five-membered ring and (4)leading to a cyclopentenenitrile derivative.
Still further, the present invention provides any one of the above production processes, wherein conjugate addition reaction is performed in the presence of an alkyl halide or an alkenyl halide, whereby alkylation or alkenylation of a γ-alkoxy-β,γ-unsaturated nitrile at the α-position thereof is performed simultaneously.
Still further, the present invention relates to use of a γ-alkoxy-β,γ-unsaturated nitrile as a starting material for the production of optically active cyclopentenenitrile derivatives. More specifically, the present invention relates to use of a 4-alkoxy-3-butenenitrile, which may have an alkyl group or an allyl group at the α-position, as a starting material for the production of cyclopentenenitrile derivatives capable of optical resolution.

The γ-alkoxy-β,γ-unsaturated nitrile to be used in the production process of the present invention includes an unsaturated nitrile, which has one or two hydrogen atoms at the α-position of nitrile, a substituent of alkoxy group at the γ-position of nitrile, and a carbon - carbon double bond at the β,γ-position of nitrile. Preferable γ-alkoxy-β,γ-unsaturated nitrile includes 4-alkoxy-3-butenenitrile which may have an alkyl group or an allyl group at the α-position (hereinafter, abbreviated as 4-alkoxy-3-butenenitriles) and the like, including, for example, a compound represented by the following general formula [1]: 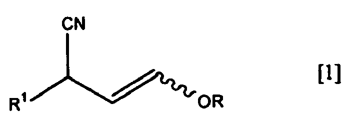 (wherein R represents alkyl group, R¹ represents hydrogen atom, alkyl group or allyl group).
In the above formula, alkyl groups represented by R and R¹ include, for example, a linear or a branched alkyl group having 1 to 20 carbon atoms, preferably 1 to 10 carbon atoms, and more preferably 1 to 6 carbon atoms, and more specifically including, for example, methyl, ethyl, propyl, isopropyl, butyl, isobutyl, sec-butyl, tert-butyl, pentyl, hexyl group, and the like.
Specific examples of the 4-alkoxy-3-butenenitriles to be used in the production process of the present invention include but obviously not limited to, for example, 4-methoxy-3-butenenitrile, 4-methoxy-2-methyl-3-butenenitrile, 2-(2-methoxyethenyl)-4-pentenenitrile, and the like.

The α,β-unsaturated ketone to be used in the production process of the present invention includes a ketone having a carbon - carbon double bond in the α,β-position thereof, and may be any of cyclic or non-cyclic, but is preferably cyclic ketones. Cyclic ketones may be monocyclic cycloalkene ring or polycyclic or fused ring of a cycloalkene ring and a cycloalkane ring. Further, the monocyclic, polycyclic or fused ring ketones may have one or more substituents such as alkyl group on the ring thereof.
The α,β-unsaturated ketone to be used in the production process of the present invention includes, for example, a cyclic ketone compound represented by the following general formula [2]: 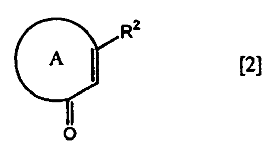 (wherein ring A represents a cycloalkene ring which may form a fused ring together with a cycloalkane ring, and R² represents hydrogen atom or alkyl group).
In the above formula, the cycloalkene ring which may form a fused ring together with a cycloalkane ring includes, for example, a cycloalkene ring or the like having 3 to 20 carbon atoms, preferably 5 to 12 carbon atoms which is monocyclic or forms a fused ring together with a cycloalkane ring.
In the above formula, the alkyl group represented by R² includes those similar to the alkyl group represented by R and R¹ described above.
In addition, the cycloalkene ring which may form a fused ring together with a cycloalkane ring represented by ring A may have one or more substitutents such as alkyl group in addition to R² on the ring thereof.

The α,β-unsaturated ketone to be used in the production process of the present invention also includes, for example, steroids having a keto group or the like.
The alkyl halide or the alkenyl halide in the process of the present invention includes an alkyl halide, which is a linear or a branched alkane compound having 1 to 20 carbon atoms, preferably 1 to 10 carbon atoms, and more preferably 1 to 6 carbon atoms, with at least one, preferably one hydrogen atom thereof being substituted by a halogen atom such as chlorine atom, bromine atom, iodine atom, or the like, or an alkenyl halide, which is a linear or a branched alkene compound having 3 to 20 carbon atoms, preferably 3 to 10 carbon atoms, and more preferably 3 to 6 carbon atoms, with at least one, preferably one hydrogen atom thereof being substituted by a halogen atom such as chlorine atom, bromine atom, iodine atom, or the like. Preferable alkyl halide or alkenyl halide includes methyl iodide, allyl bromide, or the like.
The 4-alkoxy-3-butenenitrile, which may have an alkyl group or an allyl group at the α-position in the process of the present invention, may be a compound in which an alkyl group or an allyl group has been already substituted at the α-position of cyano group. Alternatively, 4-alkoxy-3-butenenitrile can be formed in situ by using a compound not substituted (e.g. with a methylene group) at the α-position as a starting material, and performing conjugate addition reaction under basic condition in the presence of an alkyl halide or an alkenyl halide.

In the production process of the present invention, when a compound represented by the following general formula [1]: 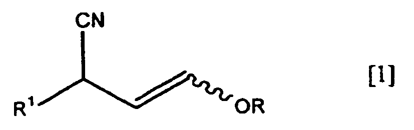 (wherein R represents alkyl group, and R¹ represents hydrogen atom, alkyl group or allyl group); is used for the 4-alkoxy-3-butenenitrile which may have an alkyl group or an allyl group at the α-position, and a compound represented by the following general formula [2]: 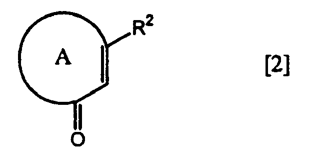 (wherein ring A represents cycloalkene ring which may form a fused ring together with a cycloalkane ring, and R² represents hydrogen atom or alkyl group); is used for the α,β-unsaturated ketone, then cyclopentenenitrile derivative to be obtained becomes a compound represented by the following general formula [3]: 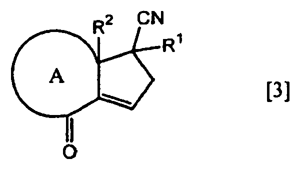 (wherein R¹ and R² are the same as described above).

In the production process of the present invention, a method thereby an enolate compound obtained by the conjugate addition reaction is esterified, then subjected to a reaction to form a five-membered ring, includes, for example, converting the resultant enolate compound to an enol ester using an esterifying agent such as a reactive derivative of carboxylic acid or carbonic acid, then treating the enol ester with a strong acid.
As the acid to be used, any of strong acids can be used, but usually, for example, mineral acids such as hydrochloric acid, sulfuric acid, perchloric acid, and the like, and organic acids such as p-toluene sulfonic acid, acetic acid, and the like are preferably used.

In the production process of the present invention, the enolate compound obtained by the conjugate addition reaction of an α,β-unsaturated ketone and a γ-alkoxy-β,γ-unsaturated nitrile is esterified and then used for the following cyclization reaction. The ester compound may be once isolated before used for the cyclization reaction, or provided for the cyclization reaction as it is as a crude product without being isolated. The method for esterifying the enolate compound can be esterifying the oxygen of the enolate moiety to form an enol ester using an esterifying agent such as various reactive derivatives of organic carboxylic acids or carbonic acid, e.g. anhydrides of carboxylic acids, acyl halides and halogenated carbonate esters. Specific examples of the esterifying agent to be used include but not limited to, for example, acetic anhydride, chlorocarbonate, and the like.

Amount of the γ-alkoxy-β,γ-unsaturated nitrile, for example, a 4-alkoxy-3-butenenitrile to be used in the production process of the present invention is generally 1 to 2 equivalents, and preferably 1.1 to 1.5 equivalents to the α,β-unsaturated ketone.
Further, amount of the esterifying agent such as acetic anhydride and chlorocarbonate to be used in the production process of the present invention is generally 1 to 3 equivalents, and preferably 1.1 to 1.5 equivalents to the α,β-unsaturated ketone.

The conjugate addition reaction by the production process of the present invention is generally conducted by stirring in a solvent such as tetrahydrofuran (THF) and cyclopentyl methyl ether, at a temperature not higher than -10°C, preferably not higher than -50°C, and more preferably not higher than -70°C, for a period of several ten minutes to several hours, in the presence of a base, under an inert gas atmosphere such as nitrogen gas and argon gas. As the base, various bases capable of generating carbanion can be used, for example, alkali metal alkoxides such as potassium alkoxide and sodium alkoxide, hydride such as sodium hydride, or the derivative thereof and the like. Preferable base includes a basic compound containing potassium.
After completion of the reaction, an esterifying agent such as acetic anhydride and chlorocarbonate is added to the reaction mixture to esterify an enolate compound as a product.
By subjecting the resultant crude product of enol ester to heat-treatment with an aqueous solution of strong acid in a solvent such as THF, hydrolysis of the enol ester moiety and intramolecular aldol condensation are promoted to give a cyclopentenenitrile derivative easily and in high yield.
When the β-position of α,β-unsaturated ketone is prochiral, the products become diastereomers and are obtained as a racemic form, but an anti-form (e.g. the methyl group and the cyano group of α,β-unsaturated ketone are in the opposite side from each other (referred to as b-form in the Examples below)) and a syn-form (e.g. the methyl group and the cyano group of α,β-unsaturated ketone are in the same side (referred to as α-form in the Examples below)) are not necessarily in a ratio of 50:50, but a compound having any one of the stereo configurations can be obtained predominantly. In the present invention, the term of "stereoselective" is used when any one of an anti-form and a syn-form is obtained predominantly as described above.
Mixture of the diastereomers obtained in the process of the present invention can be easily resolved by means of recrystallization or the like. Thus, by optically resolving a syn-form having the same stereo configuration as in CD rings of steroid, a bicyclic compound having the same stereo configuration as in CD rings of steroid can be obtained. The present inventors have found that a syn-form obtained by the process of the present invention can be easily optically resolved by means of a hydrolysis process using an enzyme. The enzyme to be used may be any enzyme so long as the enzyme can hydrolyze a carboxylate ester, preferably acetate ester, and various commercially available lipases can be used.

The production process of the present invention can be exemplified as the reaction schemes shown below using the aforementioned general formulae [1] and [2], where a potassium compound for a base and acetic anhydride for an esterifying agent are used, respectively.

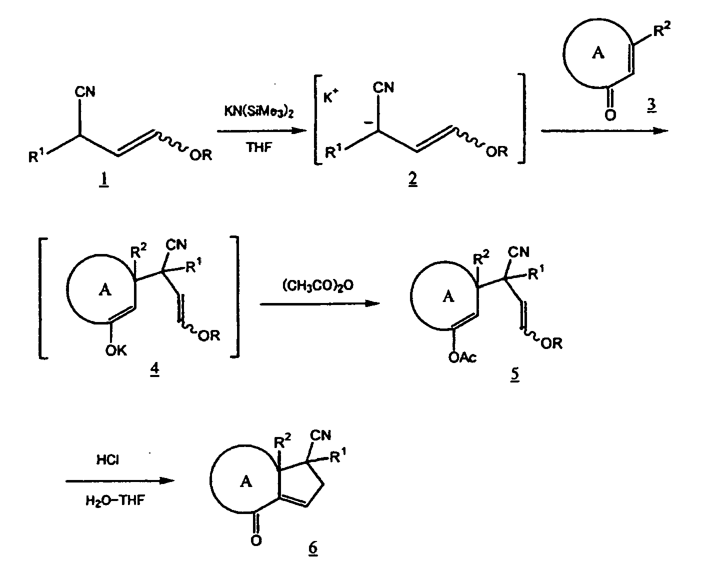

Namely, for example, 4-methoxy-3-butenenitrile (R=CH₃, R¹=H) is reacted with potassium hexamethyldisilazide (KHMDS) at a lower temperature in tetrahydrofuran, to generate carbanion 2 accompanying abstraction of a proton at the position 2.
Nitrile 1, which is an enol derivative of γ-carbonylnitrile, may exist as a mixture of a Z-form and an E-form at the carbon - carbon double bond in a ratio of about 6:4, but it is not necessary to separate the both forms.
By adding methyl iodide or allyl bromide to carbanion 2, a methyl group or an allyl group is introduced at the position 2 of nitrile 1 (R¹=CH₃ or CH₂CH=CH₂). By further reacting this compound with a base such as potassium hexamethyldisilazide (KHMDS), a carbanion is generated again by accompanying abstraction of a proton at the position 2, which can be also used for the conjugate addition reaction (for more details, see the section of Examples below).
Presence of a cyclic α,β-unsaturated ketone 3 in a solution of carbanion 2 facilitates the conjugate addition reaction to form a corresponding enolate compound 4. Addition of acetic anhydride to this reaction solution gives enol-ester 5.
In the process of the present invention, an additive to be widely used to facilitate the conjugate addition reaction such as hexamethylphosphoric triamide (HMPA) may be present, but is not necessarily required to be present. Further, even if the reaction is terminated at a lower temperature or in a shorter time, no 1,2-adduct is detected at all.
In this connection, if chlorocarbonate is used instead of acetic anhydride, a corresponding enol carbonate ester can be obtained.

Heating of enol ester 5 as it is in a mixture of tetrahydrofuran and dilute hydrochloric acid without isolating gives bicyclic enone 6 in high yield.
An enol acetate ester or an enol carbonate ester gives similar result.
Though tetrahydrofuran is used to make the reaction solution a homogeneous system, the solvent is not limited to tetrahydrofuran but any solvent other than those, which would adversely influence on the reaction, can be used.

According to the process of the present invention, cyclopentenenitrile derivatives capable of optical resolution can be easily obtained in high yield. In particular, by using cyclopentenenitrile derivatives obtained by the process of the present invention, a compound, which has a ring structure and a stereo configuration corresponding to C ring - D ring of steroid skeleton (a structure having an alkyl group such as methyl group in the β-position at the bridge head), can be produced by a simple and convenient process in high yield. For example, if the present process is applied to 3-methyl-2-cyclohexenone, a cyclopentenenitrile derivatives corresponding to the CD rings moiety of steroid can be stereoselectively obtained. These cyclopentenenitrile derivatives can be easily optically resolved. Optical resolution of the cyclopentenenitrile derivatives to produce an optically active bicyclic nitrile derivatives becomes possible, for example, by separating the diastereomers by recrystallization process or the like, reducing the keto group to hydroxyl group, which is then esterified, followed by selective hydrolysis using an enzyme such as lipase. Thus, according to the process of the present invention, a practical asymmetric synthesis of the CD rings moiety of steroid becomes possible.
The process of the present invention can be also applied to an enone, which has a large steric hindrance, and from isophorone or a Wieland-Miescher ketone, a corresponding cyclopentenenitrile derivative can be obtained in high yield, respectively.

### Effect of the Invention

The process of the present invention is characterized in that γ-alkoxy-β, γ-unsaturated nitriles having a labile hydrogen atom for abstraction at the α-position, that is, 4-alkoxy-3-butenenitriles are used as a starting material, and by using such compounds as a starting material, a reaction with an α,β-unsaturated ketone can be performed selectively and easily, and a subsequent cyclization reaction can be also performed easily and in high yield.
In the process of the present invention, a γ-alkoxy-β,γ-unsaturated nitrile as a starting material is an enol derivative of a γ-carbonylnitrile. And it has been found for the first time in the present invention that abstraction of the hydrogen atom at the α-position of cyano group occurs dominantly and selectively due to an enol derivative and a Michael reaction type of nucleophilic addition reaction proceeds.
Since the 4-alkoxy-3-butenenitriles according to the present invention respond to conjugate addition reaction with α,β-unsaturated ketones having various substitution modes in high yield, by converting these compounds to their enol esters then subjecting to a reaction to form a five-membered ring using a strong acid, various bicyclic or tricyclic cyclopentenenitrile derivatives having various substitution modes can be synthesized in only two steps in high yield. Further, since the resultant cyclopentenenitrile derivatives can be easily optically dissolved to a derivative having the same stereo configuration as the CD rings moiety of steroid, these derivatives become useful as a starting material in the synthesis of steroid skeleton. Namely, according to the process of the present invention, the CD rings moiety of steroid can be synthesized in two steps in high yield, and these can be easily optically resolved. Therefore, the process of the present invention can be led to development of a practical process for the asymmetric synthesis of the CD rings moiety of steroid.

### Best Mode for Carrying Out the Invention

Hereinafter, the present invention will be described more specifically, but by no means the present invention is limited to these Examples.

### Example 1

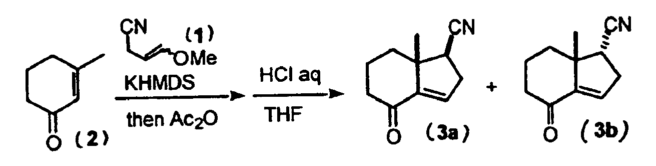

Into a 500 mL two-necked flask, potassium hexamethyldisilazide (11.0 g, 55 mmol) was charged under the argon atmosphere, and dissolved in tetrahydorofuran (200 mL) at -78°C. A solution of 4-methoxy-3-butenenitrile (1) (5.34 g, 55.0 mmol) in tetrahydrofuran (25 mL) was added thereto and the solution was stirred at -78°C for one hour. A solution of 3-methyl-2-cyclohexene-1-one (2) (5.7 mL, 50 mmol) in tetrahydrofuran (25 mL) was added and the solution was stirred at the same temperature for further one hour. Subsequently, acetic anhydride (9.4 mL, 100 mmol) was added thereto, and the temperature was raised up to -45°C over one hour before terminating the reaction with a saturated ammonium chloride aqueous solution. Two layers were separated, and the aqueous layer was extracted with ether. Organic layers were combined, washed with a saturated sodium chloride aqueous solution, dried over anhydrous magnesium sulfate, and the solvent was then evaporated off.
The resultant crude product was transferred into a 500 mL two-necked flask equipped with a reflux condenser, tetrahydrofuran (100 mL) and 3 M hydrochloric acid (100 mL) were then added thereto. After the temperature was raised up to 80°C over 30 minutes, the solution was stirred for further 2.5 hours. The reaction was terminated with a saturated sodium bicarbonate aqueous solution. The two layers were separated, and the aqueous layer was extracted with ether. The organic layers were combined, washed with a saturated sodium chloride aqueous solution, then dried over anhydrous magnesium sulfate, and the solvent was evaporated off. The resultant crude product was purified using a silica gel column chromatography (ethyl acetate / hexane = 20%), to obtain enone (3) [6.8 g, yield from enone (2): 78%, a mixture of diastereomers in a ratio of (3a):(3b) = 87:13].

### Example 2

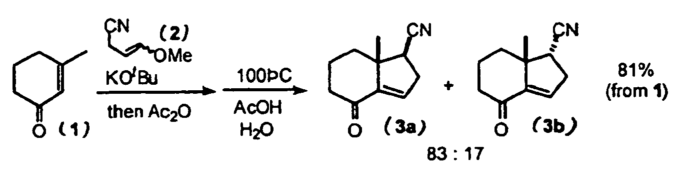

The similar procedures were repeated as in Example 1 except that potassium t-butoxide was used as a base.
Into a 100 mL two-necked flask, potassium t-butoxide (814 mg, 7.25 mmol) was charged under the argon atmosphere, and tetrahydorofuran (12.5 mL) was added thereto. The reaction mixture was then cooled down to-78°C. Subsequently, a solution of 3-methyl-2-cyclohexene-1-one (1) (0.57 mL, 5.00 mmol) and 4-methoxy-3-butenenitrile (2) (0.78 mL, 7.50 mmol) in tetrahydrofuran (4.2 mL) was added thereto. After the solution was stirred at -78°C for 30 minutes, acetic anhydride (0.95 mL, 10.0 mmol) was added thereto, and the temperature was raised up to 0°C over 10 minutes before terminating the reaction with a saturated ammonium chloride aqueous solution. The reaction mixture was extracted with ether, and the organic layer was washed with a saturated sodium chloride aqueous solution, dried over anhydrous magnesium sulfate. The solvent was evaporated off to obtain a crude product. The resultant crude product was dissolved in acetic acid (7.5 mL), transferred into 100 mL two-necked flask, and a reflux condenser was equipped thereto. After water (2.5 mL) was added thereto, and the mixture was stirred at 100°C for 8 hours, followed by evaporation of the solvent under reduced pressure. The resultant crude product was purified using a silica gel column chromatography (ethyl acetate / hexane = 20%), to obtain enone (3) [760 mg, yield: 81%, a mixture of diastereomers in a ratio of (3a):(3b) = 83:17].

### Example 3

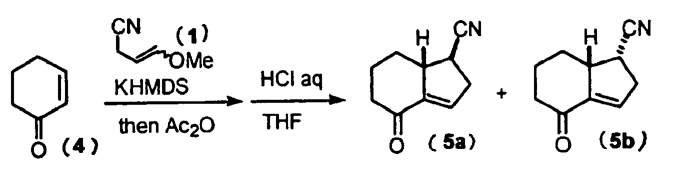

A 0.5 M solution of potassium hexamethyldisilazide in toluene (0.50 mL, 0.25 mmol) was charged into a small test tube under the argon atmosphere. After the solution was cooled down to -78°C, tetrahydrofuran (0.60 mL) was added thereto. A solution of 4-methoxy-3-butenenitrile (1) (30 mg, 0.31 mmol) in tetrahydrofuran (0.30 mL) was added thereto, and the solution was stirred at -78°C for one hour, then a solution of 2-cyclohexene-1-one (4) (22 µL, 0.23 mmol) in tetrahydrofuran (0.30 mL) was added thereto, and the solution was stirred at the same temperature for further 15 minutes. Subsequently, acetic anhydride (27 µL, 0.29 mmol) was added thereto, the temperature was then raised up to -45°C over one hour before terminating the reaction with a saturated ammonium chloride aqueous solution. The two layers were separated, and the aqueous layer was extracted with ether. The organic layers were combined, dried over anhydrous magnesium sulfate, and the solvent was then evaporated off.
The resultant crude product was transferred into a small test tube, and tetrahydrofuran (0.45 mL) and 3 M hydrochloric acid (0.45 mL) were added thereto. After the temperature was raised up to 80°C over 30 minutes, the solution was stirred for further 10 minutes. After the reaction was terminated with a saturated sodium bicarbonate aqueous solution, the two layers were separated, and the aqueous layer was extracted with ether. The organic layers were combined, dried over anhydrous magnesium sulfate, and the solvent was then evaporated off. The resultant crude product was purified using a silica gel column chromatography (ethyl acetate / hexane = 20%), to obtain enone (5) [31 mg, yield from enone (4) : 85%, a mixture of diastereomers in a ratio of (5a):(5b) = 40:60].

### Example 4

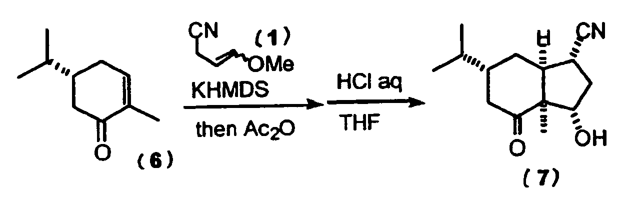

A 0.5 M solution of potassium hexamethyldisilazide in toluene (0.50 mL, 0.25 mmol) was charged into a small test tube under the argon atmosphere. After the solution was cooled down to -78°C, tetrahydrofuran (0.60 mL) was added thereto. A solution of 4-methoxy-3-butenenitrile (1) (30 mg, 0.31 mmol) in tetrahydrofuran (0.30 mL) was added thereto, and the solution was stirred at -78°C for one hour, then a solution of dihydrocarbon (6) (22 µL, 0.23 mmol) in tetrahydrofuran (0.30 mL) was added thereto, and the solution was stirred at the same temperature for further 15 minutes. Subsequently, acetic anhydride (27 µL, 0.29 mmol) was added thereto, and the solution was stirred for 30 minutes before terminating the reaction with a saturated ammonium chloride aqueous solution. The two layers were separated, and the aqueous layer was extracted with ether. The organic layers were combined, dried over anhydrous magnesium sulfate, and the solvent was then evaporated off.
The resultant crude product was transferred into a small test tube, and tetrahydrofuran (0.45 mL) and 3 M hydrochloric acid (0.45 mL) were added thereto. After the temperature was raised up to 80°C over 30 minutes, the solution was stirred for further 30 minutes. After the reaction was terminated with a saturated sodium bicarbonate aqueous solution, the two layers were separated, and the aqueous layer was extracted with ether. The organic layers were combined, dried over anhydrous magnesium sulfate, and the solvent was then evaporated off. The resultant crude product was purified using a silica gel column chromatography (ethyl acetate / hexane = 30%), to obtain hydroxyketone (7) [25 mg, yield from enone (6): 46%].

### Example 5

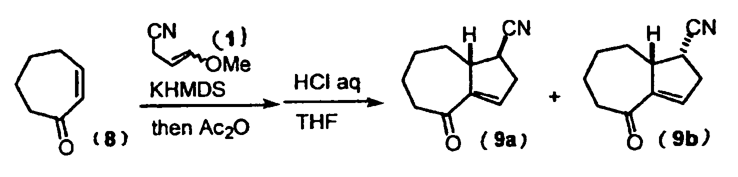

A 0.5 M solution of potassium hexamethyldisilazide in toluene (0.50 mL, 0.25 mmol) was charged into a small test tube under the argon atmosphere. After the solution was cooled down to -78°C, tetrahydrofuran (0.60 mL) was added thereto. A solution of 4-methoxy-3-butenenitrile (1) (30 mg, 0.31 mmol) in tetrahydrofuran (0.30 mL) was added thereto, and the solution was stirred at -78°C for one hour, then a solution of 2-cycloheptene-1-one (8) (25 µL, 0.23 mmol) in tetrahydrofuran (0.30 mL) was added thereto, and the solution was stirred at the same temperature for further 15 minutes. Subsequently, acetic anhydride (27 µL, 0.29 mmol) was added thereto, the temperature was then raised up to -45°C over one hour before terminating the reaction with a saturated ammonium chloride aqueous solution. The two layers were separated, and the aqueous layer was extracted with ether. The organic layers were combined, dried over anhydrous magnesium sulfate, and the solvent was then evaporated off.
The resultant crude product was transferred into a small test tube, and tetrahydrofuran (0.45 mL) and 3 M hydrochloric acid (0.45 mL) were added thereto. After the temperature was raised up to 80°C over 20 minutes, the solution was stirred for further 1.5 hours. After the reaction was terminated with a saturated sodium bicarbonate aqueous solution, the two layers were separated, and the aqueous layer was extracted with ether. The organic layers were combined, dried over anhydrous magnesium sulfate, and the solvent was then evaporated off. The resultant crude product was purified using a silica gel column chromatography (ethyl acetate /hexane = 20%), to obtain enone (9) [29 mg, yield from enone (8): 73%, a mixture of diastereomers in a ratio of (9a):(9b) = 28:72].

### Example 6

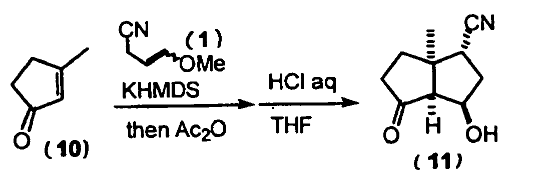

A 0.5 M solution of potassium hexamethyldisilazide in toluene (0.56 mL, 0.28 mmol) was charged into a small test tube under the argon atmosphere. After the solution was cooled down to -78°C, tetrahydrofuran (0.63 mL) was added thereto. A solution of 4-methoxy-3-butenenitrile (1) (34 mg, 0.35 mmol) in tetrahydrofuran (0.31 mL) was added thereto, the solution was stirred at -78°C for one hour, then a solution of 3-methyl-2-cyclopentene-1-one (10) (25 µL, 0.25 mmol) in tetrahydrofuran (0.31 mL) was added thereto, and the solution was stirred at the same temperature for further one hour. Subsequently, acetic anhydride (29 µL, 0.30 mmol) was added thereto, the temperature was then raised up to -55°C over one hour before terminating the reaction with a saturated ammonium chloride aqueous solution. The two layers were separated, and the aqueous layer was extracted with ether. The organic layers were combined, dried over anhydrous magnesium sulfate, and the solvent was then evaporated off.
The resultant crude product was transferred into a small test tube, and tetrahydrofuran (0.50 mL) and 3 M hydrochloric acid (0.50 mL) were added thereto. After the temperature was raised up to 80°C over 20 minutes, the solution was stirred for further 10 minutes. After the reaction was terminated with a saturated sodium bicarbonate aqueous solution, the two layers were separated, and the aqueous layer was extracted with ether. The organic layers were combined, dried over anhydrous magnesium sulfate, and the solvent was then evaporated off. The resultant crude product was purified using a silica gel column chromatography (ethyl acetate / hexane = 30%), to obtain hydroxyketone (11) [25 mg, yield from enone (10) : 92%, a mixture of diastereomers containing (11) as a main component in a ratio of 68:24:8].

### Example 7

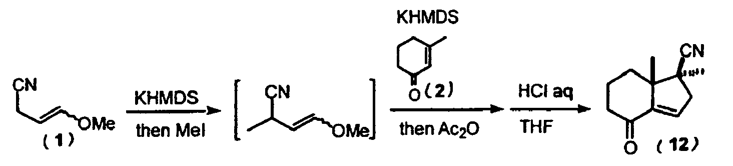

A 0.5 M solution of potassium hexamethyldisilazide in toluene (0.56 mL, 0.28 mmol) was added to a solution of 4-methoxy-3-butenenitrile (1) (27 mg, 0.28 mmol) in tetrahydrofuran (1.5 mL) at -78°C under the argon atmosphere.
After one hour, methyl iodide (17 µL, 0.28 mmol) was added thereto, and the solution was stirred at the same temperature for 30 minutes. A 0.5 M solution of potassium hexamethyldisilazide in toluene (0.44 mL, 0.22 mmol) was then added thereto, and the solution was stirred at -78°C for further one hour. Subsequently, a solution of 3-methyl-2-cyclohexene-1-one (2) (23 µL, 0.20 mmol) in tetrahydrofuran (0.50 mL) was added thereto, and the temperature was raised up to -45°C over one hour. Acetic anhydride (26 µL, 0.28 mmol) was added thereto, and the solution was stirred at the same temperature for 30 minutes before terminating the reaction with a saturated ammonium chloride aqueous solution. The two layers were separated, and the aqueous layer was extracted with ether. The organic layers were combined, dried over anhydrous magnesium sulfate, and the solvent was then evaporated off.
The resultant crude product was transferred into a small test tube, and tetrahydrofuran (0.40 mL) and 3 M hydrochloric acid (0.40 mL) were added thereto. After the temperature was raised up to 80°C over 30 minutes, the solution was stirred for further 30 minutes. After the reaction was terminated with a saturated sodium bicarbonate aqueous solution, the two layers were separated, and the aqueous layer was extracted with ether. The organic layers were combined, dried over anhydrous magnesium sulfate, and the solvent was then evaporated off. The resultant crude product was purified using a silica gel column chromatography (ethyl acetate / hexane = 20%), to obtain enone (12) [27 mg, yield from enone (2) : 71%, a mixture of diastereomers in a ratio of 91:9].

### Example 8

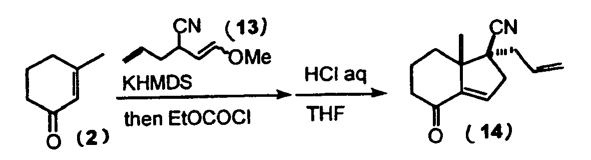

To a solution of 2-(2-methoxyethenyl)-4-pentenenitrile (13) (695 mg, 3.92 mmol) in tetrahydrofuran (11 mL) in a 100 mL two-necked flask, a 0.5 M solution of potassium hexamethyldisilazide in toluene (7.2 mL, 3.6 mmol) was added at -78°C. After the solution was stirred at -78°C for one hour, a solution of 3-methyl-2-cyclohexene-1-one (2) (0.37 mL, 3.27 mmol) in tetrahydrofuran (5.4 mmol) was added thereto, and the solution was stirred at the same temperature for 30 minutes. Subsequently, ethyl chlorocarbonate (0.44 mL, 4.58 mmol) was added, and the temperature was raised up to -45°C over one hour before terminating the reaction with a saturated ammonium chloride aqueous solution. The two layers were separated, and the aqueous layer was extracted with ether. The organic layers were combined, washed with a saturated aqueous sodium chloride solution, dried over anhydrous magnesium sulfate, and the solvent was then evaporated off.
The resultant crude product was transferred into a 50 mL two-necked flask equipped with a reflux condenser, and tetrahydrofuran (10 mL) and 3M hydrochloric acid (6.5 mL) were added thereto. After the temperature was raised up to 80°C over 15 minutes, the solution was stirred for further 1.5 hours. The reaction was terminated with a saturated sodium bicarbonate aqueous solution. The resultant two layers were separated, and the aqueous layer was extracted with ether. The organic layers were combined, washed with a saturated aqueous sodium chloride solution, dried over anhydrous magnesium sulfate, and the solvent was then evaporated off. The resultant crude product was purified using a silica gel column chromatography (ethyl acetate / hexane = 13%), to obtain enone (14) [479 mg, yield from enone (2): 68%, a mixture of diastereomers in a ratio of 95:5].

### Example 9

Synthesis of an optically active CD rings moiety of steroid using the cyclopenteneannulation reaction

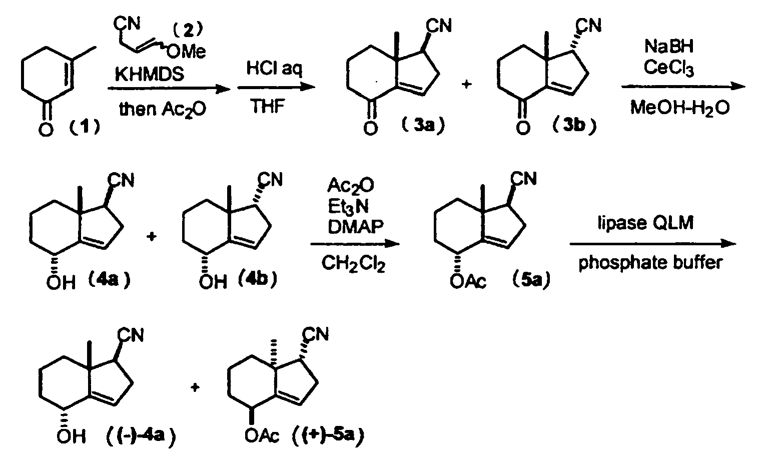

Based on the above-described synthesis scheme, bicyclic enones (3a and 3b) are synthesized from 3-methyl-2-cyclohexene-1-one (1) and 4-alkoxy-3-butenenitrile (2) by the cyclopenteneannulation reaction of the present invention, which are reduced to alcohols (4a and 4b), then esterified to acetate ester (5). Purification procedures for the intermediates are not necessary, and a diastereomer derived from (3b) can be completely removed by recrystallizing the acetate ester (5). The resultant acetate ester (5a) is hydrolyzed using an enzyme (for example, Lipase QLM: made by Meito Sangyo Co., Ltd.) to obtain an optically active alcohol ((-)-4a) and an optically active acetate ester ((+)-5a) each nearly in pure state.

### (1) Production of bicyclic enone (3)

Into a 1,000 mL two-necked flask, potassium hexamethyldisilazide (19.2 g, 91.4 mmol) was charged under the argon atmosphere. After the temperature was lowered to -78°C, tetrahydrofuran (260 mL) was added thereto. Subsequently, a solution of 4-methoxy-3-butenenitrile (28.88 g, 91.4 mmol) in tetrahydrofuran (30 mL) was added and the reaction solution was stirred for one hour, a solution of 3-methyl-2-cyclohexene-1-one (19.6 mL, 84.5 mmol) in tetrahydrofuran (50 mL) was then added thereto and the reaction solution was stirred for further one hour. Subsequently, acetic anhydride (13.0 mL, 138 mmol) was added thereto and the temperature was raised up to -45°C over one hour before terminating the reaction with a saturated ammonium chloride aqueous solution. The aqueous layer was extracted with ether, and the organic layer was combined with ether, washed with a saturated aqueous sodium chloride solution, dried over anhydrous magnesium sulfate, and the solvent was then evaporated off.
The resultant product as a solution was charged into a 1, 000 mL two-necked flask without purification, and a reflux condenser was fitted thereto. Subsequently, 3 M hydrochloric acid aqueous solution (140 mL) was added thereto, and the temperature was raised up to 80°C over 30 minutes. The solution was stirred for further 2.5 hours, then cooled down to room temperature. The reaction was terminated with a saturated sodium bicarbonate aqueous solution. The aqueous layer was extracted with ether, and the organic layer was combined with ether, washed with a saturated aqueous sodium chloride solution, dried over anhydrous magnesium sulfate, and the solvent was then evaporated off. The resultant enone (a mixture of diastereomers in a ratio of (3a):(3b) = 87:13) was used for the next reaction without purification.

### (2) Production of alcohol (4)

The crude enone produced in (1) (a mixture of diastereomers in a ratio of (3a):(3b) = 87:13) was charged into a 3,000 mL flask, and dissolved in methanol (760 mL) and water (85 mL). Cerium (III) chloride heptahydrate (47.2 g, 127 mmol) was added, the temperature was then lowered to -55°C. Sodium boron hydride (9.6 g, 254 mmol) was added, the temperature was then raised up to 0°C over one hour. The solution was diluted with water, and methanol was distilled off under the reduced pressure. After tartaric acid was added until the turbidity disappeared, the aqueous layer was extracted with ethyl acetate. The organic layer was washed with a saturated sodium chloride aqueous solution, dried over anhydrous magnesium sulfate, and the solvent was then evaporated off. The resultant alcohol (a mixture of diastereomers in a ratio of (4a):(4b) = 87:13) was used for the next reaction without purification.

### (3) Production of syn-form (5a)

The crude alcohol produced in the above (2) (a mixture of diastereomers in a ratio of (4a) : (4b) = 87:13) was charged into a 3,000 mL flask under the argon atmosphere, and dissolved in methylenechloride (280 mL) and triethylamine (35 mL, 254 mmol) . 4-Dimethylaminopyridine (1.0 g, 8.5 mmol) was added thereto, and acetic anhydride (16 mL, 169 mmol) was then added under ice cooling. After the solution was stirred for one hour, the reaction was terminated with water. The aqueous layer was extracted with ethyl acetate, and the organic layer was combined with the ethyl acetate layer, washed with a saturated sodium chloride aqueous solution, dried over anhydrous magnesium sulfate, and the solvent was then evaporated off. The resultant crude ester was dissolved in ether, and the solution was added with 3 tablespoons of activated charcoal, and then filtered through a celite layer. The filtrate was concentrated, dissolved in ethanol, and then cooled down with dry ice bath. The precipitated crystal was suction-filtered at -78°C, washed with cold ethanol to obtain crystalline acetate ester 5a (7.0 g). The mother liquor was concentrated, purified by a silica gel column chromatography (ether/hexane = 10%), recrystallized from cold ethanol to give further acetate ester 5a (3. 6 g) (total 10.6 g, overall yield through 4 steps from enone (1): 57%).

### (4) Production of optically active alcohol ((-)-4a)

A phosphate buffer was prepared by dissolving potassium dihydrogen phosphate (286 mg) and disodium hydrogen phosphate heptahydrate (2.25 g) in water (50 mL) . To a solution of acetate ester (5a) (1.10 g, 5.0 mmol) in tetrahydrofuran (5 mL), the phosphate buffer (25 mL) and Lipase QLM (made by Meito Sangyo Co., Ltd.) (250 mg) were added, and the mixture was vigorously stirred at room temperature (around 23°C) for 2 days. After sodium chloride was added to saturation, the mixture was filtered through a celite layer, and lipase remained on the celite was washed well with ether. The filtrate was washed with a saturated sodium chloride aqueous solution, dried over anhydrous magnesium sulfate, and the solvent was then evaporated off. The resultant residue was separated and purified by a silica gel column chromatography (ethyl acetate/hexane = 10% to 50%) to obtain acetate ester ((+)-5a) (543 mg, yield: 50%, optical purity: 97% ee) and alcohol ((-) -4a) (438 mg, yield: 49%, optical purity: > 99% ee). Optical purity was determined by a high performance liquid chromatography analysis using a chiral column (CHIRALCEL AS-H and OB-H, made by Daicel Chemical Industries, Ltd.).

### Industrial Applicability

The cyclopenteneannulation reaction according to the present invention enables easy production of a compound, which has the same stereo configuration as in natural products, in particular, steroid, etc., in high yield, and can be utilized to total synthesis of a natural product. Further, the process of the present invention can be effectively used as a practical process for the asymmetric synthesis of steroid.
Further, since a compound easily capable of optical resolution can be produced according to the process of the present invention, the process has an industrial applicability as a production process for various stereospecific compounds, for example, as a production process for various physiologically active substances.

## Claims

1. A process for stereospecific production of cyclopentenenitrile derivatives, **characterized in that** an α,β-unsaturated ketone and a γ-alkoxy-β,γ-unsaturated nitrile are used as starting materials.

2. The production process for cyclopentenenitrile derivatives according to claim 1, wherein the γ-alkoxy-β,γ-unsaturated nitrile is a 4-alkoxy-3-butenenitrile which may have an alkyl group or an allyl group at the α-position.

3. The production process according to claim 2, wherein the γ-alkoxy-β,γ-unsaturated nitrile is a 4-alkoxy-3-butenenitrile which may have an alkyl group or an allyl group at the α-position represented by the following general formula [1]: 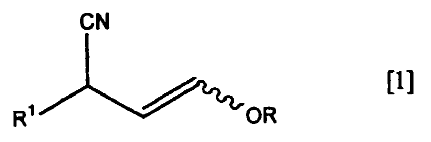 (wherein R represents alkyl group, and R¹ represents hydrogen atom, alkyl group or allyl group).

4. The production process according to claim 1, wherein the α,β-unsaturated ketone is a compound represented by the general formula [2]: 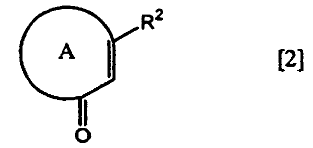 (wherein ring A represents a cycloalkene ring which may form a fused ring together with a cycloalkane ring, and R² represents hydrogen atom or alkyl group).

5. A process for production of cyclopentenenitrile derivatives, **characterized by** comprising obtaining a compound by performing conjugate addition reaction of an α,β-unsaturated ketone and a γ-alkoxx-β,γ-unsaturated nitrile, and subjecting said compound to a reaction to form a five-membered ring.

6. The process for production of cyclopentenenitrile derivatives according to claim 5, wherein the γ-alkoxy-β,γ-unsaturated nitrile is a 4-alkoxy-3-butenenitrile which may have an alkyl group or an allyl group at the α-position.

7. The production process according to claim 5 or 6, **characterized by** comprising obtaining a compound by performing conjugate addition reaction, and subjecting the compound to a reaction to form a five-membered ring without isolating said compound.

8. The production process according to claim 5 or 6, wherein the conjugate addition reaction is performed in the presence of a base containing potassium.

9. The production process according to claim 5 or 6, wherein the conjugate addition reaction is performed in the presence of an alkyl halide or an alkenyl halide, whereby alkylation or alkenylation of a γ-alkoxy-β,γ-unsaturated nitrile at the α-position thereof is performed simultaneously.

10. The production process according to claim 5 or 6, **characterized by** comprising obtaining a compound by performing conjugate addition reaction; esterifying said compound with a reactive derivative of organic carboxylic acid or carbonic acid; and thereafter subjecting said compound to a reaction to form a five-membered ring.

11. The production process according to claim 5 or 6, wherein the reaction to form a five-membered ring is a reaction to form a five-membered ring by treating with a strong acid.

12. The production process according to claim 5 or 6, **characterized by** comprising obtaining a compound by performing conjugate addition reaction, and converting said compound to an enol ester using acetic anhydride or a chlorocarbonate.

13. The production process according to claim 6, wherein the 4-alkoxy-3-butenenitrile which may have an alkyl group or an allyl group at the α-position is a compound represented by the following general formula [1]: 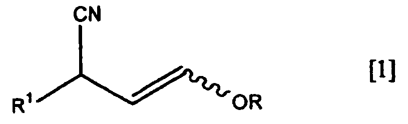 (wherein R represents alkyl group, R¹ represents hydrogen atom, alkyl group or allyl group).

14. The production process according to claim 5 or 6, wherein the α,β-unsaturated ketone is a cyclic ketone.

15. The production process according to claim 5 or 6, wherein the α,β-unsaturated ketone is steroids having a keto group.

16. The production process according to claim 5 or 6, wherein the α,β-unsaturated ketone is a compound represented by the following general formula [2]: 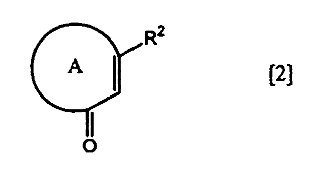 (wherein ring A represents a cycloalkene ring which may form a fused ring together with a cycloalkane ring, and R² represents hydrogen atom or alkyl group).

17. The production process according to claim 6, wherein the 4-alkoxy-3-butenenitrile which may have an alkyl group or an allyl group at the α-position is a compound represented by the general formula [1]: 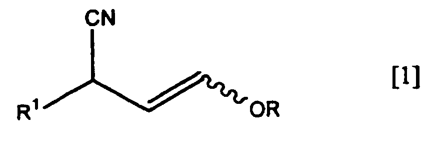 (wherein R represents alkyl group, and R¹ represents hydrogen atom, alkyl group or allyl group); the α,β-unsaturated ketone is a compound represented by the following general formula [2]: 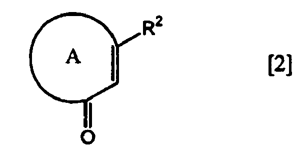 (wherein ring A represents a cycloalkene ring which may form a fused ring together with a cycloalkane ring); and the cyclopentenenitrile derivative is a compound represented by the following general formula [3]: 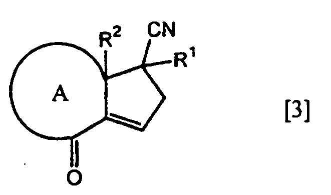 (wherein R¹ and R² are as described above).

18. The production process according to claim 17, wherein the 4-alkoxy-3-butenenitrile which may have an alkyl group or an allyl group at the α-position is 4-methoxy-3-butenenitrile.

19. Use of a γ-alkoxy-β,γ-unsaturated nitrile as a starting material for the production of cyclopentenenitrile derivatives capable of optical resolution.

20. Use according to claim 19, wherein the γ-alkoxy-β,γ-unsaturated nitrile is a 4-alkoxy-3-butenenitrile which may have an alkyl group or an allyl group at the α-position.

21. A production process for optically active alcohol derivatives comprising producing a cyclopentenenitrile derivative by the production process according to any one of claims 1 to 18; reducing a keto group of said cyclopentenenitrile derivative to obtain a corresponding alcohol derivative; and thereafter subjecting the alcohol derivative to optical resolution thereto.

22. The process according to claim 21, wherein the optical resolution is an optical resolution process using an enzyme.
